# EUROPÄISCHE PATENTSCHRIFT

(11) **EP 3 759 065 B1**
(45) Veröffentlichungstag und Bekanntmachung des Hinweises auf die Patenterteilung: **03.08.2022**
(21) Anmeldenummer: 19710577.8
(22) Anmeldetag: 28.02.2019
(51) Int. Cl.: C07C 29/60, C07C 31/10, B01J 35/10, B01J 37/02, B01J 23/30, B01J 23/652, B01J 27/188, B01J 37/00

(54) **VERFAHREN ZUR KATALYTISCHEN UMWANDLUNG VON GLYCERIN IN PROPANOL**
METHOD FOR THE CATALYTIC CONVERSION OF GLYCEROL TO PROPANOL
PROCÉDÉ POUR LA TRANSFORMATION CATALYTIQUE DE GLYCÉRINE EN PROPANOL

(30) Priorität: 28.02.2018 AT 501732018
(43) Veröffentlichungstag der Anmeldung: 06.01.2021
(73) Patentinhaber: OMV REFINING & MARKETING GMBH, 1020 Wien (AT)
(72) Erfinder: SCHÖFFL, Paul, 4210 Gallneukirchen (AT)
(74) Vertreter: SONN Patentanwälte OG
(86) Internationale Anmeldenummer: PCT/AT2019/060065
(87) Internationale Veröffentlichungsnummer: WO 2019/165486

(56) Entgegenhaltungen:
- WO-A1-2013/163561
- CN-B- 103 706 392
- WANG ET AL.: "Catalytic transformation of glycerol to 1-propanol by combining zirconium phosphate and supported Ru catalysts", RCS ADVANCES, Bd. 6, 16. März 2016 (2016-03-16), Seiten 29769-29777, XP002790322,
- XUFENG LIN ET AL: "Hydrogenolysis of Glycerol by the Combined Use of Zeolite and Ni/Al 2 O 3 as Catalysts: A Route for Achieving High Selectivity to 1-Propanol", ENERGY & FUELS., Bd. 28, Nr. 5, 15. Mai 2014 (2014-05-15), Seiten 3345-3351, XP055428009, WASHINGTON, DC, US. ISSN: 0887-0624, DOI: 10.1021/ef500147k

## Beschreibung

Die Erfindung betrifft ein Verfahren und eine Vorrichtung zur katalytischen Umwandlung einer Stoffmischung enthaltend Glycerin in einem Festbettreaktor in Propanole, wobei Substrate des Katalysators anorganische Materialien und/oder Metalloxide aufweisen. Weiters betrifft die Erfindung einen Katalysator und ein Verfahren zur Herstellung eines Katalysators zur katalytischen Umwandlung einer Stoffmischung enthaltend Glycerin in Propanole in einem Festbettreaktor, wobei Substrate des Katalysators anorganische Materialien und/oder Metalloxide aufweisen.

Die Nachfrage nach Energie und insbesondere Brennstoffen steigt seit langem aufgrund der Industrialisierung und Modernisierung. Aus diesem Grund wurde, beispielsweise von der Europäischen Union, eine Vielzahl an Richtlinien erlassen um den Ausstoß von Treibhausgasen durch fossile Brennstoffe zu reduzieren. Dabei werden fossile Brennstoffe teilweise durch erneuerbar produzierte Brennstoffe ersetzt. Dies wird beispielsweise in der EU durch die nunmehr novellierte Version der erneuerbaren Energien Richtlinie (RED II) geregelt, die vorschreibt, dass ein Anteil von 14 Energieprozent aus nachwachsenden Rohstoffen stammen muss. Ein weiteres Ziel ist die Beimengung von fortschrittlichen Biokraftstoffen (advanced biofuels) im Ausmaß von Energieprozent 0.2e% ab 2022, 1e% ab 2025 und 3,5e% 2030 zu Kraftstoffen im Transportsektor, wobei diese nur aus einer eingeschränkten Liste von Rohstoffen, welche in der Richtlinie festgelegt sind, hergestellt werden dürfen. Der Vorteil fortschrittlicher Biokraftstoffe ist, dass diese zu keinem direkten Wettbewerb mit der Nahrungsmittelproduktion und somit auch nur zu reduzierten indirekten Landnutzungsänderungen (ILUC) führen. Derzeit gibt es nur wenige kommerzielle Verfahren für die Produktion dieser Komponenten. Viele der Prozesse werden erst in kleinen Maßstäben getestet und daher sind Komponenten dieser Art teuer und nur schwer und in nicht ausreichender Menge zu erwerben.

Rohglycerin ist gemäß der Direktive der EU ein gelistetes Ausgangsmaterial. Aus der Biodieselproduktion fallen etwa 10 Gewichtsprozent Glycerin als Koppelprodukt in Form von Rohglycerin an. Rohglycerin ist prozessbedingt üblicherweise mit Natriumchlorid, Wasser, Methanol, Sulfaten, Monoglyceriden, Diglyceriden, Asche, unzureichend abgetrennten Fettrückständen und anderen Stoffen verunreinigt. Unter dem Begriff Rohglycerin im Sinne der Erfindung wird ein flüssiges Gemisch mit einem Glycerinanteil von wenigstens 60 Gew.-% verstanden, das zusätzlich auch einen Mineralstoffanteil, d.h. einen Anteil an Kationen wie Natrium, Kalium, Calcium, Magnesium sowie Chlor und Phosphor, von 1 Gew.-% bis 10 Gew.-% und einen Wasseranteil von mehr als 5 Gew.-% aufweist. U. a. auf Grund des günstigen Preises, der flüssigen Beschaffenheit und der guten Mengenverfügbarkeit hat Rohglycerin ein großes Potential signifikant zur Herstellung fortschrittlicher Biokraftstoffe beizutragen. Aus energetischer Sicht am vorteilhaftesten ist, aus Glycerin (Propan-1,2,3-triol) auf Grund der Stöchiometrie die entsprechenden Mono-Alkohole, d.h. 1-Propanol (n-Propanol) und/oder 2-Propanol (Isopropanol) zu produzieren. Zudem wird auf Grund der höheren Energiedichte fortschrittlicher Bio-Propanole als Beimischungskomponente verhältnismäßig weniger benötigt als bei fortschrittlichem Bio-Ethanol. Der vielversprechendste Ausgangsstoff zur Produktion von fortschrittlichen Bio-Propanolen ist Rohglycerin als Nebenprodukt der Biodieselherstellung.

Viele verschiedene Prozesse zur Umwandlung von Glycerin in hochwertige bzw. nützliche Chemikalien wurden bereits getestet und analysiert, wie die Oxidierung, die Veresterung und die Hydrogenolyse von Glycerin.

So zeigt die US 8,507,736 B2 ein Verfahren zur Erzeugung von kurzkettigen Alkoholen aus Glycerin, welches als Nebenprodukt der Biodieselproduktion erzeugt wird. Im Einzelnen kann der Produktstrom eine Mischung aus Ethanol, Methanol und Propanol umfassen, wobei Propanol mehr als 50 Prozent der Gesamtmasse der monohydrischen Alkohole im Produktstrom ausmacht. Die Verarbeitung kann in einem einzigen Reaktionsschritt erfolgen, der sowohl Dehydratation als auch Hydrierung umfasst. Der dabei eingesetzte Katalysator kann eine Mischung eines Dehydratationskatalysator und einem Hydrierungskatalysator sein, wobei ersterer Oxide von Wolfram und Zirkonium enthalten kann und letzterer ein Metall aus der Platingruppe enthalten kann. Die Reaktion in einem einzigen Schritt soll bei etwa 300 °C erfolgen.

Weiters offenbart die US 8,946,458 B2 eine Reihe von Katalysatoren und Verfahren und Reaktorsystemen zur Verarbeitung sauerstoffhaltiger Kohlenwasserstoffe. Als Einsatz wird insbesondere auf die Verwendung von Glycerin als Nebenprodukt aus der Biodieselproduktion hingewiesen. Die daraus gewonnenen Produkte umfassen unter anderem 1-Propanol und Aceton. Für die Reaktion wird ein heterogener Hydrodeoxygenierungskatalysator (HDO) verwendet, wobei eine Vielzahl an unterschiedlichen Katalysatoren beschrieben sind. Hinsichtlich der Dotierung wird erwähnt, dass ein bimetallischer Katalysator mit Platin und Molybdän auf einem Wolfram-Zirkoniumträger verwendet werden kann. Als Reaktionstemperatur wird ein Bereich zwischen 100 °C und 300 °C bei einem Druck von umgerechnet zwischen 4 und 140 bar angegeben.

Die EP 2 016 037 B1 zeigt ein Verfahren zur Verarbeitung eines wässrigen Einsatzes, der auch Glycerin umfasst, in einem einzigen katalytischen Prozess und unter der Verwendung von in-situ erzeugtem Wasserstoff unter anderem zu Alkoholen, welche insbesondere Ethanol und Propanol umfassen. Als Katalysatorträger kann Silizium oder Zirkonium verwendet werden, welche mit Wolfram behandelt sein können. In diesem Zusammenhang wird erwähnt, dass die Verwendung von Oxiden unter anderem von Zirkonium bevorzugt verwendet wird. Der Katalysator kann außerdem ein Übergangsmetall, z.B. Platin oder Rhodium, enthalten. Das Verfahren läuft in zwei chemischen Schritten ab, für die unterschiedliche - allerdings überlappende - Temperaturbereiche von zwischen 80 °C und 400 °C bzw. zwischen 100 °C und 300 °C angegeben sind.

Die CN 101054339 D4 zeigt ein Verfahren zur Verarbeitung von dem Biodiesel-Nebenprodukt Glycerin zu n-Propanol in einem kontinuierlichem Prozess mit einem Festbettkatalysator mit mehreren Rohren. Als Katalysatorträger wird u. a. ZrO2 verwendet, wobei u. a. Wolfram, Rhodium, Molybdän und Platin als aktive Komponenten zur Anwendung kommen. Die Reaktionstemperatur beträgt zwischen 180 und 360 °C.

Weitere Verfahren sind beispielsweise in US 8,075,642 B2 und DE 10 2008 026 583 A1 offenbart.

Alle derzeit verfügbaren Verfahren weisen allerdings einen oder mehrere essenzielle Nachteile auf. So ist es üblicherweise nur möglich, raffiniertes Glycerin, aber nicht Rohglycerin oder verunreinigtes Glycerin direkt zu verarbeiten. Um aus Rohglycerin raffiniertes Glycerin zu erhalten, müssen Verunreinigungen entfernt werden, wobei allerdings nicht jedes produzierte Glycerin zu raffiniertem Glycerin weiterverarbeitet werden kann. Häufig wird als einziger Reinigungsschritt Methanol aus dem Rohglycerin zurückgewonnen. Aufgrund des ausfallenden umfangreichen Reinigungsprozesses ist Rohglycerin weit günstiger als raffiniertes Glycerin. Insbesondere sind keine Katalysatoren oder Verfahren zur Herstellung solcher Katalysatoren bekannt, die diese Anforderungen zufriedenstellend erfüllen, wodurch ein kontinuierlicher Betrieb verhindert wird. So können sich bei den Techniken des Stands der Technik beispielsweise Salze oder Schwefel auf dem Katalysator ablagern und dessen Wirkung somit mindern. Weiters sind alle Verfahren komplex, haben eine geringe Selektivität oder einen geringen Umsatz und/oder sind teuer durchzuführen.

Ein Ziel der Erfindung ist somit alle oder manche der oben angeführten Nachteile zu beheben und insbesondere ein Verfahren und eine Vorrichtung sowie einen Katalysator und ein Verfahren zur Herstellung eines Katalysators zu schaffen, mit dem sowohl der Umsatz von Glycerin als auch die Selektivität zum Produkt Propanol hoch ausfallen. Weiters ist es ein Ziel, Rohglycerin ohne aufwendige Vorreinigung oder Aufbereitung zu einem Treibstoff oder einer zumischbaren Treibstoffkomponente weiterzuverarbeiten. Ein Ziel ist weiters, die katalytisch aktiven Verbindungen am Substrat des Katalysators zu immobilisieren, um zu vermeiden, dass diese häufig leicht wasserlöslichen katalytisch aktiven Substanzen im Laufe des Prozesse herausgelöst werden. Weiters soll es möglich sein, die vorgeschlagenen Verfahren vom Labor auf die Großanlage in ausreichender Dimensionierung zu übertragen, d.h. dass es entsprechendes Upscaling erlauben sollte. Weiters sollen Bedingungen erreicht werden, unter denen die Produktion von Nebenprodukten, wie beispielsweise Dimerisationsprodukten und Ketonen, limitiert wird.

Dies wird erreicht durch ein Verfahren und eine Vorrichtung zur Umwandlung einer Stoffmischung enthaltend Glycerin in Propanole wie eingangs beschrieben, wobei die Substrate des Katalysators einen Porendurchmesser an der Oberfläche zwischen 10 und 25 Ängström, bevorzugt zwischen 12 und 20 Ängström, besonders bevorzugt im Wesentlichen von 15 Ängström aufweisen. Weiters wird dies erzielt durch einen Katalysator zur Umwandlung einer Stoffmischung enthaltend Glycerin in Propanole wie eingangs beschrieben, wobei die Substrate des Katalysators einen Porendurchmesser an der Oberfläche zwischen 10 und 25 Ängström, bevorzugt zwischen 12 und 20 Ängström, besonders bevorzugt im Wesentlichen von 15 Ängström aufweisen und durch ein Verfahren zur Herstellung eines Katalysators zur Umwandlung einer Stoffmischung enthaltend Glycerin in Propanole wie eingangs beschrieben, wobei Grundmaterialien zur Herstellung des Substrates des Katalysators einen Porendurchmesser an der Oberfläche zwischen 10 und 25 Ängström, bevorzugt zwischen 12 und 20 Ängstrom, besonders bevorzugt im Wesentlichen von 15 Ängstrom aufweisen oder das Grundmaterial zur Herstellung der Substrate des Katalysators USY-Zeolithe aufweist, wobei die USY-Zeolithe dealuminiert werden, sodass die Substrate des Katalysators einen Porendurchmesser an der Oberfläche zwischen 10 und 25 Ängström, bevorzugt zwischen 12 und 20 Ängstrom, besonders bevorzugt im Wesentlichen von 15 Ängstrom aufweisen. Die genannten Porendurchmesser können auch im Inneren der Substrate oder einem Teilbereich desselben vorgesehen sein.

Untersuchungen haben gezeigt, dass durch diese Herstellungsmethode und durch diese Auswahl eines Substrates mit solchen Porengrößen die ansonsten üblicherweise gut wasserlöslichen katalytisch aktiven Substanzen, beispielsweise Silicowolframsäure, gut am Katalysatorsubstrat immobilisiert werden. Das heißt, dass beispielsweis bei einer Reaktion die in flüssiger, wässriger Phase z.B. unter einem Druck von 25 bis 50 bar der Katalysator, insbesondere der Dehydratationskatalysator nicht durch Hydratation, d. h. Herauslösung mit Wasser, gelöst werden kann, sondern stabil immobilisiert bleibt und somit die aktiven Zentren trotz wässriger Reaktionsführung frei zugänglich und erhalten bleiben.

Vorteilhafterweise soll die Reaktion in einem einzigen Schritt erfolgen. Frühere Forschungen haben nahegelegt, dass ein kombiniertes Katalysatorsystem notwendig ist, da bei der Reaktion auf die Dehydratation eine Hydrierung folgen muss. Somit sind vorteilhafterweise zwei verschiedene Katalysatorsysteme auf einem Substrat kombiniert, damit die Reaktion in einem einzigen Schritt erfolgen kann. Die Immobilisierung der katalytisch aktiven Substanzen, insbesondere des Dehydratationskatalysators, beispielsweise Silicowolframsäure, erfolgt höchstwahrscheinlich einerseits auf Grund physikochemischer Wechselwirkungen, vermutlich adsorptiv wirkender Van-der-Waals-Kräfte, und andererseits der idealen Porendurchmesser und somit der Unzugänglichkeit der katalytisch aktiven Substanz. Weiters erfolgt die Abscheidung der katalytisch aktiven Substanz, insbesondere des Dehydratationskatalysators, auf Grund der Molekülgröße vorzugsweise oberflächennah, wobei die Abscheidung insbesondere in groben Poren erfolgt, und im Unterschied zum Hydrierungskatalysator der Dehydratationskatalysator nicht weiter in das Substrat eindringen kann.

Bezugnehmend auf das Verfahren zur Umwandlung einer Stoffmischung enthaltend Glycerin in Propanole ist es vorteilhaft, wenn die Substrate des verwendeten Katalysators Extrudat-Pellets und vorzugsweise keramisch sind. Durch eine Extrudierung und anschließende Konditionierung von Katalysator-Pellets verflüchtigen sich vorteilhafterweise organische Bindemittel an der Substratoberfläche beinahe rückstandslos und die Pellets erhalten eine entsprechende Festigkeit.

Bevorzugt erfolgt die Umwandlung der Stoffmischung enthaltend Glycerin in Propanole mit dem vorhin beschriebenen Katalysator im Festbettreaktor kontinuierlich. Auf diese Weise kann ein möglichst hoher Glycerin Durchsatz gefahren und gleichzeitig ein sehr hoher Umsatz zu Propanolen von über 85% erzielt werden. Auf Grund der Immobilisierung des ansonsten wasserlöslichen Dehydratisierungskatalysators, ermöglicht durch die speziellen Substrate des Katalysators, bleibt die Katalysatoraktivität langfristig auf diesem hohen Niveau erhalten und Katalysatorwechselintervalle und damit Standzeiten der Anlage werden auf ein Minimum reduziert. Vorzugsweise ist der Festbettreaktor einstufig.

In einer bevorzugten Variante des Verfahrens werden als Substrat des Katalysators Mischoxide aus Silizium, Wolfram, Zirkonium und/oder Aluminium, vorzugsweise Zirkondioxid, Zeolithe (vorzugsweise synthetische Alumosilikate und Alumophosphate), bevorzugt VFI- oder VPI-5-Zeolithe, dealuminierte USY-Zelithe und/oder Aluminiumdioxid verwendet. Solche Substrate stellten sich im Laufe von Versuchsserien als besonders günstig heraus.

Ebenfalls ist es vorteilhaft, wenn der Katalysator eine Platindotierung aufweist. Das Immobilisieren von Platin als zweite katalytisch aktive Substanz des Katalysators am Substrat stellt laut dem Stand der Technik kein Problem dar. Nach aktuellem Stand der Technik ist ZrO2 kein vorteilhafter Katalysator für die Umwandlung von Glycerin in Propanole, allerdings ist es als Trägermaterial für den Katalysator in vielen Reaktionen aufgrund seiner hohen thermischen Stabilität, großen Härte und guten Stabilität unter reduzierenden Bedingungen vorteilhaft. Es sind vermutlicherweise Säurezentren für den Dehydratationsschritt notwendig, in dem Acetol und 3-Hydroxypropionaldehyd die möglichen Produkte aus Glycerin sind. Phosphorwolframsäure könnte ebenfalls eine Möglichkeit für den Dehydratationsschritt sein. Vorteilhafterweise ist ein Metallkatalysator für die Hydrierung der Zwischenprodukte verantwortlich. Edelmetalle wie beispielsweise Platin sind bekannt dafür, Hydrierkatalysatoren zu sein, vermutlich da sie Wasserstoffmoleküle aktivieren. Möglich ist ebenfalls Ruthenium, da es nicht so empfindlich auf Schwefel reagiert wie andere Katalysatoren. Weiters sind unedle Metalle wie beispielsweise Kupfer oder Nickel möglich. Im Allgemeinen sollte der Katalysator ein optimales Verhältnis aus Säurezentren und Hydrierzentren aufweisen und vorteilhafterweise möglichst resistent gegen Katalysatorvergiftung sein. Vorzugsweise besteht die Zusammensetzung des Katalysators neben dem Substrat aus einem Anteil an Platin, Ruthenium, Kupfer oder Nickel von 0,5 bis 5 Gew.-Prozent, bevorzugt zwischen 1 und 3 Gew.-Prozent, besonders bevorzugt im Wesentlichen von 1,5 Gew.-Prozent sowie aus Silicowolframsäure oder Phosphorwolframsäure zwischen 5 bis 20 Gew.-Prozent bevorzugt zwischen 7.5 und 15 Gew.-Prozent, besonders bevorzugt im Wesentlichen von 12 Gew.-Prozent.

Als Ausgangsmaterial, d.h. als Stoffmischung enthaltend Glycerin, können Rohglycerin, technisches Glycerin und/oder gereinigtes Glycerin verwendet werden. Besonders vorteilhaft ist es, wenn Rohglycerin verwendet wird, da dieses sich durch einen niedrigen Preis auszeichnet und bei verschiedenen Prozessen als Nebenprodukt anfällt. Weiters zählt üblicherweise nur dieses zu den (gesetzlichen) Zielvorgaben bezüglich fortschrittlicher Biokraftstoffe, insbesondere gemäß Richtlinie (EU) 2015/1513, Anhang IX, Teil A auch bekannt als "RED II". Technisches Glycerin liegt in Bezug auf den Glyceringehalt und den Anteil an Verunreinigungen zwischen Rohglycerin und gereinigtem Glycerin.

Vorzugsweise wird in einem ersten Schritt des Verfahrens die Stoffmischung enthaltend Glycerin aus einem Vorlagebehälter entnommen und vorteilhafterweise filtriert, insbesondere um je nach Qualität der Stoffmischung enthaltend Glycerin, insbesondere je nach Grad der Verunreinigung, eventuell darin enthaltene ungelöste Feststoffe sowie unerwünschte Begleitstoffe zu entfernen. Fakultativ kann die Stoffmischung enthaltend Glycerin vor oder nach diesem Schritt zentrifugiert werden. Bevorzugt anschließend wird die (vorzugsweise vorbehandelte) Stoffmischung enthaltend Glycerin mit Wasser gemischt und verdünnt, wobei vorzugsweise je nach gewünschter Produktausbeutestruktur der Glyceringehalt auf zwischen 5 und 80 Prozent, besonders bevorzugt auf zwischen 10 und 60 Prozent, noch mehr bevorzugt auf zwischen 15 und 50 Prozent eingestellt wird, wobei der Glyceringehalt auf das 0,7-fache bis 0,9-fache der gewünschten, stöchiometrisch möglichen Konzentration (Stoffmenge) von 1-Propanol im Endprodukt eingestellt wird. Sofern dieser Schritt durchgeführt wird, bezeichnet im Folgenden die Stoffmischung enthaltend Glycerin die in diesem Schritt erzielte Mischung der ursprünglichen (vorzugsweise vorbehandelten) Stoffmischung enthaltend Glycerin mit Wasser.

In einem vorteilhaften Verfahrensschritt wird die Stoffmischung enthaltend Glycerin vorzugsweise über einen Wärmetauscher, bevorzugt einen Economiser zur quasi-adiabatischen Reaktionsführung bevorzugt auf zwischen 150 und 300 °C, besonders bevorzugt auf zwischen 190 und 250 °C, noch mehr bevorzugt auf im Wesentlichen 220 °C erhitzt. Vorzugsweise wird die Stoffmischung enthaltend Glycerin, bevorzugt in einem statischen Mixer, mit Wasserstoff versetzt, wobei bevorzugt Wasserstoff im Überschuss auf das 10-fache bis 60-fache der gewünschten, stöchiometrisch möglichen Konzentration (Stoffmenge) von 1-Propanol im Endprodukt eingestellt wird.

Sofern dieser Schritt durchgeführt wird, bezeichnet die Stoffmischung enthaltend Glycerin (zusätzlich zu ggf. vorhergegangenen Zusätzen und Reinigungen) die mit Wasserstoff gemischte Stoffmischung.

Anschließend wird die Stoffmischung enthaltend Glycerin über einen, vorzugsweise kontinuierlichen, katalytischen Festbettreaktor geschickt. Dabei wird vorzugsweise das Prozessfluid (die umgesetzte Stoffmischung enthaltend Glycerin) nach dem Festbettreaktor zur Wärmeeinbringung zum Wärmetauscher geführt. Die Energie aus der Prozesswärme reicht vorteilhafterweise aus um die Vorheizung der Stoffmischung enthaltend Glycerin vor der Umsetzung im Festbettreaktor zur Reaktion zu gewährleisten, es muss also (ausgenommen beim Anfahren der Anlage) keine externe Reaktionsenergie zugeführt und/oder abgeführt werden, die Reaktionsführung erhält sich somit vorteilhafterweise adiabatisch.

Anschließend wird vorteilhafterweise das Prozessmedium entspannt, bevorzugt auf das zwischen 0,2 und 0,02-fache , besonders bevorzugt auf das zwischen 0,1 und 0,03-fache, noch mehr bevorzugt auf im Wesentlichen das 0,04-fache des ursprünglichen Drucks. Dann wird bevorzugt Wasserstoff aus der umgesetzten Stoffmischung über eine Abscheideeinrichtung rückgewonnen, wobei der rückgewonnen Wasserstoff über einen Kompressor rekomprimiert wird und dem Frischwasserstoff, der der Stoffmischung vor der Umsetzung im Festbettreaktor zugesetzt wurde, beigemengt. Dabei beträgt der Anteil des rückgewonnenen Wasserstoffs am Gesamtwasserstoffeinsatz zur Reaktion bevorzugt zwischen 50 und 99 Prozent, besonders bevorzugt zwischen 70 und 97 Prozent.

In weiteren vorteilhaften Verfahrensschritten kann von der umgesetzten Stoffmischung, bevorzugt physikalisch, besonders bevorzugt destillativ, eine organische Zielfraktion gewonnen werden, d.h. dass das enthaltene sowie als Koppelprodukt entstandene Prozesswasser entfernt wird und Reinpropanole gewonnen werden. Vorzugsweise werden sowohl 1-Propanol als auch 2-Propanol gewonnen. Der Produktstrom enthält vorzugsweise zwischen 10 und 22 Gew.-% 2-Propanol und zwischen 60 und 85 Gew.-% 1-Propanol, bspw. 20 Gew.-% 2-Propanol und 70 Gew.-% 1-Propanol. Es kann allerdings auch nur eines der beiden gewonnen werden. Bevorzugt erfolgt die Gewinnung von 1-Propanol und/oder 2-Propanol über eine Aufbereitungskaskade, indem Wasser aus dem im Festbettreaktor umgesetzten Stoffgemisch zunächst entfernt wird und Reinpropanole gewonnen werden, wobei das abgetrennte Prozesswasser einem Frischwasser in einem Wassermischer, das ist üblicherweise ein gewöhnlicher Mischer, beigemengt wird und die Wassermischung besonders bevorzugt in dem Verfahren an einer früheren Stelle, insbesondere zur Mischung mit dem Gemisch enthaltend Glycerin im Mischer, zum Einsatz kommt, wobei noch mehr bevorzugt das rückgewonnene Prozesswasser einen Anteil zwischen 80 und 100 Prozent, vorzugsweise zwischen 90 und 100 Prozent des am gesamten im Verfahren eingesetzten Wassers hat. Es kann auch alternativ oder zusätzlich die gewonnene organische Zielfraktion in einem weiteren Verfahrensschritt dazu verwendet werden, längerkettige Kohlenwasserstoffe aufzubauen, d.h. zu oligomerisieren.

Vorzugsweise wird die Umsetzung im Festbettreaktor bei einer Temperatur zwischen 150 und 300 °C, bevorzugt zwischen 190 und 250 °C, besonders bevorzugt zwischen 210 und 230 °C durchgeführt. Experimente haben gezeigt, dass die Umwandlung bei niedrigeren Temperaturen gering war, wohingegen hohe Temperaturen eine geringere Selektivität für Propanole bedingte, wobei bei den genannten Temperaturintervallen, insbesondere beim Intervall von 210 bis 230 °C, eine gute Effizienz in Bezug auf das optimale Verhältnis zwischen Energieaufwand, Konversion und Selektivität war.

In einer bevorzugten Variante wird die Umsetzung im Festbettreaktor bei einem Druck zwischen 10 und 100 bar, bevorzugt zwischen 15 und 75 bar, noch mehr bevorzugt zwischen 25 und 50 bar durchgeführt, da unter diesen Bedingungen ebenfalls eine besonders gute Effizienz erreicht werden kann.

Der sich aus dem erfindungsgemäßen Verfahren ergebende Produktstrom weist insbesondere zwischen 0,1 und 5 Gew.-% MeOH, zwischen 3 und 6 Gew.-% EtOH, zwischen 10 und 22 Gew.-% 2-Propanol, zwischen 3 und 6 Gew.-% Aceton und zwischen 60 und 85 Gew.-% 1-Propanol auf. Es wird vorzugsweise ein Glyceringehalt im Produkt von kleiner 0,05 Gew.-% erreicht.

In einem Ausführungsbeispiel wurde Rohglycerin verwendet, dessen chemische Analyse die folgende Zusammensetzung zeigte:
Na: 0,13 Gew.-%
Ca: <0,01 Gew.-%
K: 0,61 Gew.-%
Al: <0,001 Gew.-%
Si: <0,001 Gew.-%
Fe: 0,0004 Gew.-%
Cr: <0,0001 Gew.-%
Ni: <0,0001 Gew.-%
S: 0,5548 Gew.-%
Cl: <0,0001 Gew.-%
V: <0,001 Gew.-%
Zn: <0,001 Gew.-%
Kohlenstoff: 32,30 Gew.-%
Wasserstoff: 9,80 Gew.-%
Wasser: 14,0 Gew.-%
Glyceringehalt: 79,9 Gew.-%
Säurezahl: 0,45 mg KOH/g

Der Prozess wurde in diesem Ausführungsbeispiel mit einer WHSV (Weight Hourly Space Velocity) von 0,80 pro h, einem Verhältnis von Wasserstoff zu Glycerin von 33 mol/mol, einer mittleren Reaktionstemperatur von 220 °C, wobei über den Reaktor verteilt die Temperatur zwischen 217 und 225 °C lag, und einem Druck von 50 bar durchgeführt. Daraus ergab sich die folgende Produktzusammensetzung:
MeOH: 0,3 Gew.-%
EtOH: 5,6 Gew.-%
2-Propanol: 13,9 Gew.-%
Aceton: 5,5 Gew.-%
1-Propanol: 74,6 Gew.-%

Dabei wurden Spuren anderer Analyten, anorganische Bestandteile sowie Wasser im Rahmen einer Extraktivdestillation entfernt.

Die Konversion ist abhängig von der WHSV bzw. dem molaren Verhältnis von Wasserstoff zu Glycerin und der Temperatur. Beispielhaft werden die folgenden Konversionen erreicht:

**Beispiel 1: WHSV: 0,07 pro h; Verhältnis Wasserstoff zu Glycerin: 133 mol/mol**

| | | | |
|---|---|---|---|
| Temperatur [°C] | 200 | 225 | 250 |
| Konversion [%] | 100 | 100 | 100 |

**Beispiel 2: WHSV: 0,07 pro h; Verhältnis Wasserstoff zu Glycerin: 66 mol/mol**

| | | | |
|---|---|---|---|
| Temperatur [°C] | 200 | 225 | 250 |
| Konversion [%] | 75 | 100 | 100 |

**Beispiel 3: WHSV: 0,15 pro h; Verhältnis Wasserstoff zu Glycerin: 32 mol/mol**

| | | | |
|---|---|---|---|
| Temperatur [°C] | 200 | 225 | 250 |
| Konversion [%] | 58 | 99 | 100 |

**Beispiel 4: WHSV: 0,21 pro h; Verhältnis Wasserstoff zu Glycerin: 33 mol/mol**

| | | | |
|---|---|---|---|
| Temperatur [°C] | 200 | 225 | 250 |
| Konversion [%] | 22 | 80 | 96 |

Die Konversion (in %) wurde berechnet als (Glycerin (eingang) - Glycerin (ausgang))/Glycerin (ausgang)*100. Höhere Temperaturen begünstigen höhere Konversionen. Ebenso begünstigen höhere molare Verhältnisse höhere Konversionen. Das Glycerin kann zu über 80 Gew.-% in Propanole umgesetzt werden, bevorzugt zu über 90 Gew.-%.

Bezugnehmend auf die erfindungsgemäße Vorrichtung zur katalytischen Umwandlung einer Stoffmischung enthaltend Glycerin in einem Festbettreaktor in Propanole ist es vorteilhaft, wenn die Substrate des Katalysators vorzugsweise keramische Extrudat-Pellets sind.

Weiterhin ist es vorteilhaft, wenn die Substrate des Katalysators Mischoxide aus Silizium, Wolfram, Zirkonium und/oder Aluminium, vorzugsweise Zirkondioxid, Alumosilikat- und/oder Alumophosphat-Zeolithe, vorzugsweise VFI- und/oder VPI-5-Zeolithe, dealuminierte USY-Zelithe und/oder Aluminiumdioxid sind. Ebenfalls vorteilhaft ist es, wenn das Substrat eine Platindotierung aufweist. Weiters bevorzugt ist der Festbettreaktor zum kontinuierlichen Betrieb geeignet.

Die Vorrichtung weist vorzugsweise eines oder mehrere der folgenden Objekte auf:
- einen Vorlagebehälter für die Stoffmischung enthaltend Glycerin,
- eine Filtervorrichtung zur Entfernung von in der Stoffmischung enthaltend Glycerin enthaltenen, ungelösten Feststoffen und/oder unerwünschten Begleitstoffen,
- einen ersten Mischer zur Verdünnung der Stoffmischung enthaltend Glycerin mit Wasser,
- einen Wärmetauscher, vorzugsweise einen Economiser, zur Erhitzung der Stoffmischung enthaltend Glycerin,
- einen vorzugsweise statischen zweiten Mischer zur Versetzung der Stoffmischung enthaltend Glycerin mit Wasserstoff,
- eine Leitung zur Rückführung der im Festbettreaktor umgesetzten Stoffmischung zum Wärmetauscher,
- eine Abscheideeinrichtung zur Rückgewinnung von Wasserstoff aus der im Festbettreaktor umgesetzten Stoffmischung und vorzugsweise einen Kompressor zum Komprimieren des rückgewonnenen Wasserstoffs, der einem Ausgangswasserstoff zugeführt und mit dieser vermischt werden kann,
- eine Aufbereitungskaskade 11, welche mittels Extraktionsmittel die Reinpropanole und das Prozesswasser trennt, wobei die Extraktionsmittel für den Extraktionskreislauf in der Aufbereitungskaskade rückgewonnen werden und vorzugsweise auch das Prozesswasser rückgewonnen wird, vorteilhafterweise für die erneute Verwendung zur Herstellung einer verdünnten Stoffmischung enthaltend Glycerin im Mischer.

Bezugnehmend auf den erfindungsgemäßen Katalysator zur katalytischen Umwandlung einer Stoffmischung enthaltend Glycerin in Propanole in einem Festbettreaktor ist es vorteilhaft, wenn die Substrate, vorzugsweise keramische, Extrudat-Pellets sind. Weiters ist vorteilhaft, wenn die Substrate des Katalysators Mischoxide aus Silizium, Wolfram, Zirkonium und/oder Aluminium, vorzugsweise Zirkondioxid, Alumosilikat und/oder Alumophosphat Zeolithe, bevorzugt VFI- und/oder VPI-5-Zeolithe, dealuminierte USY-Zelithe und/oder Aluminiumdioxid und vorzugsweise eine Platindotierung aufweisen und/oder der Katalysator zum kontinuierlichen Betrieb des Festbettreaktors geeignet ist.

Bezugnehmend auf das erfindungsgemäße Verfahren zur Herstellung eines Katalysators zur katalytischen Umwandlung einer Stoffmischung enthaltend Glycerin in Propanole in einem Festbettreaktor ist es wesentlich, dass die Substrat-Grundmaterialien bereits eine geeignete Porengröße, insbesondere einen Porendurchmesser an der Oberfläche zwischen 10 und 25 Ängström, bevorzugt zwischen 12 und 20 Ängstrom, besonders bevorzugt im Wesentlichen von 15 Ängstrom aufweisen, sodass schlussendlich der Porendurchmesser an der Oberfläche der Substrate an deren Oberfläche bei zwischen 10 und 25 Ängström, bevorzugt zwischen 12 und 20 Ängstrom, besonders bevorzugt im Wesentlichen bei 15 Ängstrom liegt. Sofern der Porendurchmesser der Grundmaterialien noch nicht im entsprecheden Bereich liegt, wie dies bei USY-Zeolithen der Fall ist, ist es notwendig, den Porendurchmesser durch geeignete Maßnahmen, insbesondere durch Dealuminierung, zuerst auf eine geeignete Größe zu bringen. USY-Zeolithe sind sogenannte ultra-stabilisierte (Y-)Zeolithe. Diese haben eigentlich eine zu kleine Porengröße, sind jedoch mechanisch extrem stabil. Diese Eigenschaft ist insbesondere im Up-Scaling, beispielsweise aufgrund der hohen Reaktoren und der Druckbeanspruchung durch das Eigengewicht, relevant. Wenn diese USY-Zeolithe dealuminiert werden, d.h. ein definierter Teil des Alumosilikatverbundes nachträglich chemisch entfernt wird, kann die Porengröße passend erweitert werden, insbesondere auf den genannten gewünschten Porendurchmesser an der Oberfläche des Substrats. Die Porenweite, die mechanische Stabilität und das Potential für Oberflächenwechselwirkungen bzw. die Physisorption zur Immobilisierung beispielsweise der Silicowolframsäure sind zusammenfassend die entscheidenden Kriterien zur Auswahl des Substratmaterials. Bezugnehmend auf die Verwendung von USY-Zeolithen, die ursprünglich noch nicht die richtige Porengröße aufweisen, als Grundmaterial für die Herstellung der Substrate des Katalysators, ist es vorteilhaft, wenn die Dealuminierung mit einem Komplexierungsmittel, insbesondere mit EDTA, erfolgt.

Es ist es vorteilhaft, wenn als Grundmaterialien für die Substrate (das Supportmaterial) des Katalysators Mischoxide aus Silizium, Wolfram, Zirkonium und/oder Aluminium, vorzugsweise Zirkondioxid, Alumosilikat und/oder Alumophosphat Zeolithe, bevorzugt VFI- und/oder VPI-5-Zeolithe, dealuminierte USY-Zelithe und/oder Aluminiumdioxid verwendet werden.

Vorzugsweise werden zunächst organische Bindemittel in Grundmaterialien, die bevorzugt pulverförmig vorliegen, eingearbeitet, diese homogen zu einer Grundmasse verknetet und die organischen Bindemittel im Anschluss im Wesentlichen rückstandslos, insbesondere im Wesentlichen an der Substratoberfläche rückstandslos, ausgebrannt. Als organische Bindemittel wird bevorzugt eine Dispersion, besonders bevorzugt ein Nano-Dispersion, aus Polymeren, vorzugsweise Polystyrol, und Wasser verwendet werden, wobei bevorzugt der Polymeranteil kleiner als 5 Gewichtsprozent, besonders bevorzugt kleiner als 1 Gewichtsprozent an der Dispersion beträgt. Anschließend werden aus der Grundmasse bevorzugt unter einem Druck zwischen 5 und 120 bar, besonders bevorzugt zwischen 20 und 100 bar, noch mehr bevorzugt zwischen 40 und 80 bar auf einen Durchmesser zwischen 1 und 8 mm, bevorzugt zwischen 2 und 6 mm, besonders bevorzugt zwischen 3 und 4 mm und/oder eine Länge zwischen 0,25 und 4 cm, bevorzugt zwischen 0,4 und 3 cm, besonders bevorzugt zwischen 0,5 und 2 cm Pellets extrudiert und vorzugsweise die Pellets bei einer Temperatur zwischen 400 und 1000 °C, bevorzugt zwischen 500 und 750 °C, noch mehr bevorzugt bei im Wesentlichen 600 °C für einen Zeitraum zwischen 24 und 168 h, bevorzugt zwischen 36 und 96 h, noch mehr bevorzugt für im Wesentlichen 48h konditioniert.

Weiters ist es vorteilhaft, wenn die extrudierten Substrat-Pellets mit Platin imprägniert werden, wobei pro 1000 g Substratmaterial vorzugsweise zwischen 0,5 und 3 Litern, bevorzugt im Wesentlichen 1,5 Liter wässriger Lösung von zwischen 15 und 300 mmol/l, bevorzugt im Wesentlichen 75 mmol/1 H2PtCl6·6H2O zur Imprägnierung verwendet werden. Dann werden die Pellets bevorzugt getrocknet, wobei dies bei einer Temperatur zwischen 60 und 120 °C, vorzugsweise im Wesentlichen 90 °C durchgeführt wird. Anschließend werden die Pellets vorteilhafterweise bei zwischen 250 und 450 °C, bevorzugt im Wesentlichen 350 °C für zwischen 6 und 24 Stunden, bevorzugt im Wesentlichen 12 Stunden kalziniert.

Weiters vorteilhaft ist eine anschließende Imprägnierung des platindotierten Substrats mit zwischen 0,5 und 3 Litern, bevorzugt im Wesentlichen 1,5 Litern zwischen 25 und 100 mmol/l, bevorzugt im Wesentlichen 50 mmol/l wässriger Silicowolframsäure zur oberflächlichen Fixierung des Dehydratationskatalysators, vorzugsweise anschließende Trocknung bei zwischen 60 und 120 °C, vorzugsweise im Wesentlichen 90 °C und vorzugsweise anschließende Kalzinierung bei zwischen 250 und 450 °C, bevorzugt im Wesentlichen 350 °C für zwischen 6 und 24 Stunden, bevorzugt im Wesentlichen 12 Stunden.

Durch diese Herstellungsmethode und die Auswahl von Substraten mit der zuvor definierten Porengröße haben Untersuchungen gezeigt, dass ansonsten gut wasserlösliche Katalysatoren, insbesondere Dehydratationskatalysatoren, wie beispielsweise Silicowolframwsäure, am Katalysatorsubrat immobilisiert werden. Das heißt, dass bei der Reaktion die in flüssiger, wässriger Phase und unter dem zuvor genannten Druck ausgeführt wird, der Dehydratationskatalysator nicht durch Hydratation gelöst werden kann, sondern stabil immobilisiert bleibt und weiters die aktiven Zentren dabei frei zugänglich bleiben. Die Immobilisierung des Dehydratationskatalysators, insbesondere von Silicowolframsäure, erfolgt höchstwahrscheinlich auf Grund physikochemischer Wechselwirkungen einerseits (vermutlich adsorptiv wirkender van-der-Waals-Kräft) und des idealen Porendurchmessers und somit der Unzugänglichkeit des Dehydratationskatalysators für Hydratation andererseits.

Eine alternative Möglichkeit stellt die Co-Extrusion des Dehydratationskatalysators, beispielsweise Silicowolframoxiden, direkt mit in das Supportmaterial, beispielsweise Zircondioxid, dar, sodass in der Folge für eine spätere Imprägnierung keine Rücksicht auf den Porendurchmesser genommen werden muss. Diese Alternative hat sich allerdings als sehr aufwändig und komplex herausgestellt.

Die Erfindung wird nachstehend anhand von einem in der Zeichnung dargestellten bevorzugten Ausführungsbeispiel, auf welche sie jedoch keinesfalls beschränkt sein soll, noch näher erläutert. Im Einzelnen zeigt die Zeichnung:

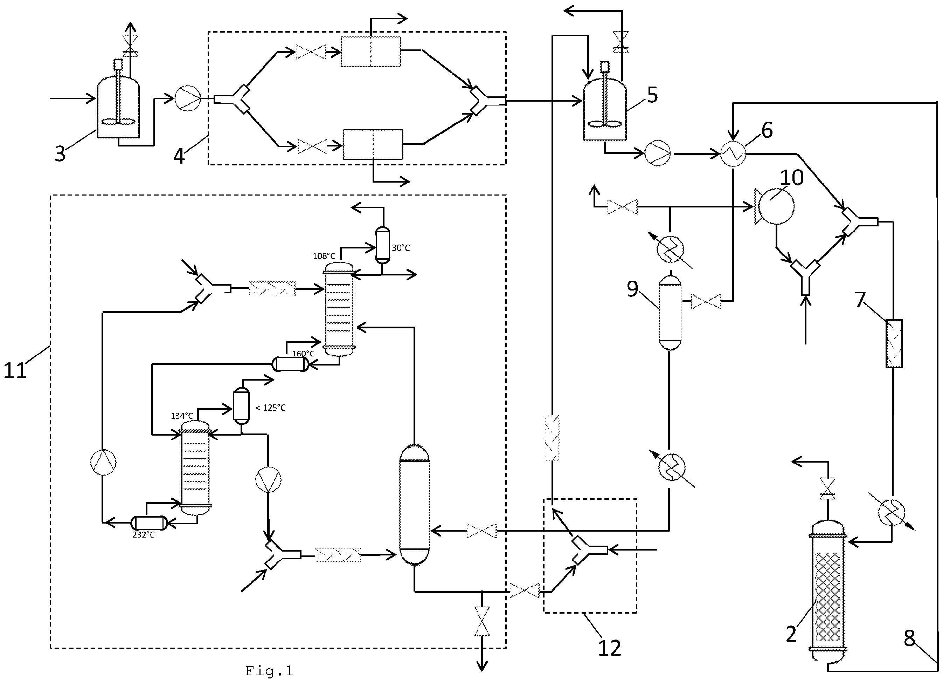

Fig. 1 eine bevorzugte Ausführungsform des Verfahrens und der Vorrichtung zur katalytischen Umwandlung einer Stoffmischung enthaltend Glycerin in Propanole in einem Festbettreaktor.

Fig. 1 zeigt eine bevorzugte Ausführungsform der Vorrichtung 1 zur katalytischen Umwandlung einer Stoffmischung enthaltend Glycerin in Propanole in einem Festbettreaktor 2. Als Ausgangsmaterial wird insbesondere Rohglycerin verwendet, dass einem Vorlagebehälter 3 entnommen wird. Anschließend wird die Stoffmischung enthaltend Glycerin, vorzugsweise mit einer Pumpe, einer Filtervorrichtung 4 zugeführt, in der sie filtriert wird um je nach Qualität der Stoffmischung enthaltend Glycerin eventuell darin enthaltene, ungelöste Feststoffe sowie unerwünschte Begleitstoffe zu entfernen. Im Konkreten Ausführungsbeispiel besteht die Filtervorrichtung aus zwei redundant angeordneten, rückspülbaren Filtern, wobei vor dem jeweiligen rückspülbaren Filter vorzugsweise ein Regelventil angeordnet ist. Anschließend wird die filtrierte Stoffmischung enthaltend Glycerin in einem Mischerbehälter 5 mit Wasser gemischt und verdünnt, wobei der resultierende Glyceringehalt vorzugsweise zwischen 10 und 60 Prozent liegt. Der Mischerbehälter 5 weist dabei ein Sicherheitsventil (Überdruckventil) auf.

Anschließend wird die filtrierte und verdünnte Stoffmischung enthaltend Glycerin über einen Economiser-Wärmetauscher 6 erhitzt, dem die Stoffmischung enthaltend Glycerin über eine Pumpe zugeführt wurde, und anschließend im statischen Mixer 7 mit Wasserstoff versetzt und vorteilhafterweise anschließend über einen Kühler geführt. Dann wird diese Mischung dem kontinuierlichen, katalytischen Festbettreaktor 2 zugeführt, der wiederum vorzugsweise ein Sicherheitsventil aufweist. Die umgesetzte Stoffmischung wird zur Wärmeeinbringung im Wärmetauscher 6 über die Leitung 8 geschickt. Dabei reicht zur Vorheizung der filtrierten und vermischten Stoffmischung enthaltend Glycerin die Energie der Prozessabwärme aus und es muss üblicherweise keine externe Reaktionsenergie zugeführt werden. Somit erhält sich die Reaktionsführung adiabat. Eine Ausnahme davon liegt selbstverständlich während des Einschaltvorganges vor.

Anschließend wird das umgewandelte Stoffgemisch über ein Flashventil einer Abscheideeinrichtung 9, insbesondere ein Dampf-Flüssigkeit-Abscheider bzw. eine Flash-Drum, zur Rückgewinnung von Wasserstoff aus der im Festbettreaktor umgesetzten Stoffmischung zugeführt. Der Wasserstoff-Recyclegasstrom wird über einen Luftkühler geleitet und leichtflüchtige organische Begleitkomponenten rückkondensieren in den Abscheidebehälter. In der Folge wird ein Teil des Wasserstoff-Recyclegasstroms zum Ausbringen von Koppelproduktgasen (insbesondere vorwiegend Propan) nach einem Regelventil abgefackelt , wobei der Anteil des abgefackelten Wasserstoff-Recyclegasstroms unter 0,5% beträgt und besonders bevorzugt unter 0,1% beträgt. Anschließend wird der rückgewonnene Wasserstoff in einem Kompressor 10 rekomprimiert und dem Frischwasserstoff beigemengt, mit dem die Stoffmischung enthaltend Glycerin im statischen Mixer 7 vermischt wird, wobei der Anteil des recycelten Wasserstoffs am Wasserstoffeinsatz in einem Zyklus vorzugsweise zwischen 70 und 97 Prozent beträgt. Aus der umgesetzten Stoffmischung können in einer Aufbereitungskaskade 11, der die umgesetzte Stoffmischung über einen Wärmetauscher und einen Kühler zugeführt wird, anschließend die Reinpropanole gewonnen sowie die Extraktionsmittel für den Extraktionskreislauf in selbiger Aufbereitungskaskade rückgewonnen werden und auch das Prozesswasser für die erneute Verwendung zur Herstellung einer verdünnten Stoffmischung enthaltend Glycerin rückgewonnen wird, wobei das rückgewonnene Prozesswasser in einem Wassermischer 12 mit Frischwasser vermengt und anschließend im Mischer 5 dem Stoffgemisch enthaltend Glycerin zugesetzt wird. Bevorzugt beträgt der Anteil des rückgewonnen Prozesswasser am gesamten Wassereinsatz eines Zyklus zwischen 80 und 100 Prozent, noch mehr bevorzugt zwischen 90 und 100 Prozent.

Das der Aufbereitungskaskade 11 zugeführte umgesetzte Stoffgemisch läuft zuerst über ein Regelventil und wird einer Abscheidevorrichtung zugeführt, der als Extraktionsmittel Toluol beigeführt wird und in der das Prozesswasser vom umgesetzten Stoffgemisch getrennt wird, das anschließend wie bereits erläutert über ein Regelventil dem Wassermischer 12 zugeführt wird, wobei über ein weiteres Regelventil Schmutzwasser abgeführt wird. Weiters wird von der Abscheidevorrichtung eine Mischung aus Propanolen und Toluol einer ersten Extraktionskolonne zugeführt, der als Extraktionsmittel N-Methyl-2-pyrrolidon (NMP) beigeleitet wird. In dieser wird das Kopfprodukt enthaltend die Zielfraktion (Propanole) bei 108°C abgezogen und anschließend werden über eine Kondensationskolonne bei 30°C die Propanole gewonnen. Weiters wird aus der ersten Exktraktionskolonne eine Mischung aus Toluol und NMP, beispielsweise bei einer Temperatur von 160°C, über eine Verdampfungskolonne ausgeschieden und einer zweiten Extraktionskolonne, beispielsweise bei 134°C, zugeführt. In dieser zweiten Extraktionskolonne wird das Sumpfprodukt abgezogen und das Resttoluol abdestilliert und der zweiten Extraktionskolonne zur Energieeinbringung wieder zugeführt, und in der Folge das nicht verdampfte NMP bei etwa 232 °C über eine Pumpe - mit frischem NMP in einem statischen Mixer vermengt - der ersten Extraktionskolonne zur Ausscheidung der Propanole wiederum zugeführt. In der zweiten Extraktionskolonne wird ebenfalls das Kopfprodukt abgezogen und das Toluol bei unter 125 °C auskondensiert und über eine Pumpe und in einem statischen Mixer vermischt mit frischem Toluol der Abscheidevorrichtung als Extraktionsmittel beigeführt.

Das in der Figur dargestellte und im Zusammenhang mit dieser erläuterte Ausführungsbeispiel dient der Erläuterung der Erfindung und ist für diese nicht beschränkend. Die im Ausführungsbeispiel angeführten Temperaturen sind ausschließlich beispielhaft zu verstehen, andere Temperaturen sind möglich.

## Patentansprüche

1. Verfahren zur katalytischen Umwandlung einer Stoffmischung enthaltend Glycerin in Propanole in einem Festbettreaktor (2), wobei Substrate des Katalysators anorganische Materialien und/oder Metalloxide aufweisen, **dadurch gekennzeichnet, dass** die Substrate einen Porendurchmesser an der Oberfläche zwischen 10 und 25 Ängström, bevorzugt zwischen 12 und 20 Ängström, besonders bevorzugt von 15 Ängström aufweisen, wobei der Katalysator Silicowolframsäure oder Phosphorwolframsäure aufweist.

2. Verfahren nach Anspruch 1, **dadurch gekennzeichnet, dass** die Substrate Extrudat-Pellets und vorzugsweise keramisch sind; und/oder
**dadurch gekennzeichnet, dass** die Umsetzung der Stoffmischung enthaltend Glycerin im Festbettreaktor (2), der vorzugsweise einstufig ist, kontinuierlich erfolgt.

3. Verfahren nach einem der Ansprüche 1 oder 2, **dadurch gekennzeichnet, dass** als Substrat des Katalysators Mischoxide aus Silizium, Wolfram, Zirkonium und/oder Aluminium, vorzugsweise Zirkondioxid, Alumosilikat- und/oder Alumophosphat-Zeolithe, bevorzugt VFI- und/oder VPI-5-Zeolithe, dealuminierte USY-Zelithe und/oder Aluminiumdioxid verwendet werden, wobei bevorzugt der Katalysator eine Platindotierung aufweist; und/oder
**dadurch gekennzeichnet, dass** die Stoffmischung enthaltend Glycerin Rohglycerin, technisches Glycerin oder gereinigtes Glycerin ist.

4. Verfahren nach einem der vorhergehenden Ansprüche, **dadurch gekennzeichnet, dass** das Verfahren einen oder mehrere der folgenden Schritte aufweist:
das Entnehmen der Stoffmischung enthaltend Glycerin aus einem Vorlagebehälter (3),
Filtrierung der Stoffmischung enthaltend Glycerin, wobei vorzugsweise darin enthaltene, ungelöste Feststoffe und/oder verdünnte Begleitstoffe entfernt werden, und/oder
Mischung der Stoffmischung enthaltend Glycerin mit Wasser im Mischer (5), wobei bevorzugt die Konzentration des Glycerins auf zwischen 5 und 80 Prozent, besonders bevorzugt auf zwischen 10 und 60 Prozent, noch mehr bevorzugt auf zwischen 15 und 50 Prozent eingestellt wird; und/oder
**dadurch gekennzeichnet, dass** das Verfahren einen oder mehrere der folgenden Schritte aufweist:
Erhitzung der Stoffmischung enthaltend Glycerin mit einem Wärmetauscher (6), vorzugsweise einem Economiser,
Versetzung der Stoffmischung enthaltend Glycerin mit Wasserstoff, vorzugsweise in einem statischen Mixer (7),
Rückführen der Wärme der im Festbettreaktor (2) umgesetzten Stoffmischung zum Wärmetauscher (6) zum Wärmeübertrag an die Stoffmischung enthaltend Glycerin vor der Umsetzung, sodass das Verfahren adiabatisch erfolgt.

5. Verfahren nach einem der vorhergehenden Ansprüche, **dadurch gekennzeichnet, dass** die im Festbettreaktor (2) umgesetzte Stoffmischung über eine Abscheideeinrichtung (9) Wasserstoff aus der im Festbettreaktor (2) umgesetzten Stoffmischung rückgewonnen wird, wobei vorzugsweise der rückgewonnene Wasserstoff anschließend in einem Kompressor (10) rekomprimiert und einem Frischwasserstoff beigemengt wird, wobei die Wasserstoffmischung besonders bevorzugt in dem Verfahren an einer früheren Stelle zum Einsatz kommt und noch mehr bevorzugt der rückgewonnene Wasserstoff einen Anteil zwischen 50 und 99 Prozent, vorzugsweise zwischen 70 und 97 Prozent des am gesamten im Verfahren eingesetzten Wasserstoff hat; und/oder
**dadurch gekennzeichnet, dass**
von dem im Festbettreaktor (2) umgesetzten Stoffgemisch Propanole, vorzugsweise sowohl 1-Propanol als auch 2-Propanol, gewonnen werden, bevorzugt indem Wasser aus dem im Festbettreaktor (2) umgesetzten Stoffgemisch über eine Aufbereitungskaskade (11) zunächst entfernt wird und Reinpropanole gewonnen werden, wobei das abgetrennte Prozesswasser einem Frischwasser in einem Wassermischer (12) beigemengt wird und die Wassermischung besonders bevorzugt in dem Verfahren an einer früheren Stelle, insbesondere zur Mischung im Mischer (5), zum Einsatz kommt und noch mehr bevorzugt das rückgewonnene Prozesswasser einen Anteil zwischen 80 und 100 Prozent, vorzugsweise zwischen 90 und 100 Prozent des am gesamten im Verfahren eingesetzten Wassers hat und/oder
aus dem im Festbettreaktor (2) umgesetzten Stoffgemisch längerkettige Kohlenwasserstoffe aufgebaut, vorzugsweise oligomerisiert, werden.

6. Verfahren nach einem der vorhergehenden Ansprüche, **dadurch gekennzeichnet, dass** die Umsetzung im Festbettreaktor (2) bei einer Temperatur zwischen 150 und 300 °C, bevorzugt zwischen 190 und 250 °C, besonders bevorzugt zwischen 210 und 230 °C erfolgt; und/oder
**dadurch gekennzeichnet, dass** die Umsetzung im Festbettreaktor (2) bei einem Druck von 10 und 100 bar, bevorzugt 15 und 75 bar, besonders bevorzugt zwischen 25 und 50 bar erfolgt.

7. Vorrichtung (1) zur katalytischen Umwandlung einer Stoffmischung enthaltend Glycerin, vorzugsweise Rohglycerin, in Propanole in einem Festbettreaktor (2), wobei die Vorrichtung einen Katalysator aufweist und Substrate des Katalysators anorganische Materialien und/oder Metalloxide aufweisen, **dadurch gekennzeichnet**, die Substrate einen Porendurchmesser an der Oberfläche zwischen 10 und 25 Ängström, bevorzugt zwischen 12 und 20 Ängström, besonders bevorzugt im Wesentlichen von 15 Ängström aufweisen, wobei die Substrate bevorzugt Extrudat-Pellets und vorzugsweise keramisch sind, wobei
der Katalysator Silicowolframsäure oder Phosphorwolframsäure aufweist.

8. Vorrichtung (1) nach Anspruch 7, **dadurch gekennzeichnet, dass**
die Substrate des Katalysators Mischoxide aus Silizium, Wolfram, Zirkonium und/oder Aluminium, vorzugsweise Zirkondioxid, Alumosilikat- und/oder Alumophosphat-Zeolithe, bevorzugt VFI- und/oder VPI-5-Zeolithe, dealuminierte USY-Zelithe und/oder Aluminiumdioxid und vorzugsweise eine Platindotierung aufweisen, wobei bevorzugt der Festbettreaktor (2) zum kontinuierlichen Betrieb geeignet ist.

9. Vorrichtung (1) nach einem der Ansprüche 7 oder 8, **dadurch gekennzeichnet, dass** die Vorrichtung aufweist:
einen Vorlagebehälter (3) für die Stoffmischung enthaltend Glycerin,
eine Filtervorrichtung (4) zur Entfernung von in der Stoffmischung enthaltend Glycerin enthaltenen, ungelösten Feststoffen und/oder unerwünschten Begleitstoffen,
einen ersten Mischer (5) zur Verdünnung der Stoffmischung enthaltend Glycerin mit Wasser,
einen Wärmetauscher (6), vorzugsweise einen Economiser, zur Erhitzung der Stoffmischung enthaltend Glycerin,
einen vorzugsweise statischen zweiten Mischer (7) zur Versetzung der Stoffmischung enthaltend Glycerin mit Wasserstoff,
eine Leitung (8) zur Rückführung der im Festbettreaktor umgesetzten Stoffmischung zum Wärmetauscher,
eine Abscheideeinrichtung (9) zur Rückgewinnung von Wasserstoff aus der im Festbettreaktor (2) umgesetzten Stoffmischung und vorzugsweise einen Kompressor (10) zum Komprimieren des rückgewonnenen Wasserstoffs, und/oder
eine Aufbereitungskaskade (11) zur Gewinnung der Reinpropanolfraktion sowie vorzugsweise zur Rückgewinnung des Prozesswassers aus der im Festbettreaktor (2) umgesetzten Stoffmischung zur Mischung mit Frischwasser im Wassermischer (12), wobei die dabei gewonnene Wassermischung vorzugsweise dem Mischer (5) zugeführt wird.

10. Katalysator zur katalytischen Umwandlung einer Stoffmischung enthaltend Glycerin in Propanole in einem Festbettreaktor (2), wobei Substrate des Katalysators anorganische Materialien und/oder Metalloxide aufweisen, **dadurch gekennzeichnet, dass** die Substrate einen Porendurchmesser an der Oberfläche zwischen 10 und 25 Ängström, bevorzugt zwischen 12 und 20 Ängström, besonders bevorzugt im Wesentlichen von 15 Ängström aufweisen, wobei
der Katalysator Silicowolframsäure oder Phosphorwolframsäure aufweist.

11. Katalysator nach Anspruch 10, **dadurch gekennzeichnet, dass** die Substrate Extrudat-Pellets und vorzugsweise keramisch sind, wobei
die Substrate des Katalysators bevorzugt Mischoxide aus Silizium, Wolfram, Zirkonium und/oder Aluminium, vorzugsweise Zirkondioxid, Alumosilikat- und/oder Alumophosphat-Zeolithe, bevorzugt VFI- und/oder VPI-5-Zeolithe, dealuminierte USY-Zelithe und/oder Aluminiumdioxid und vorzugsweise eine Platindotierung aufweisen und/oder
der Katalysator zum kontinuierlichen Betrieb des Festbettreaktors (2) geeignet ist.

12. Verfahren zur Herstellung eines Katalysators nach einem der Ansprüche 10 oder 11 zur katalytischen Umwandlung einer Stoffmischung enthaltend Glycerin in Propanole in einem Festbettreaktor, wobei Substrate des Katalysators anorganische Materialien und/oder Metalloxide aufweisen, **dadurch gekennzeichnet, dass** Grundmaterialien zur Herstellung der Substrate des Katalysators einen Porendurchmesser an der Oberfläche zwischen 10 und 25 Ängström, bevorzugt zwischen 12 und 20 Ängstrom, besonders bevorzugt im Wesentlichen von 15 Ängstrom aufweisen, und dass das Substrat mit Silicowolframsäure oder Phosphorwolframsäure versehen wird.

13. Verfahren nach Anspruch 12, **dadurch gekennzeichnet, dass** als Grundmaterialien für die Substrate Mischoxide aus Silizium, Wolfram, Zirkonium und/oder Aluminium, vorzugsweise Zirkondioxid, Alumosilikat und/oder Alumophosphat Zeolithe, bevorzugt VFI- und/oder VPI-5-Zeolithe, und/oder Aluminiumdioxid verwendet werden.

14. Verfahren zur Herstellung eines Katalysators nach einem der Ansprüche 10 oder 11 zur katalytischen Umwandlung einer Stoffmischung enthaltend Glycerin in Propanole in einem Festbettreaktor, wobei Substrate des Katalysators anorganische Materialien und/oder Metalloxide aufweisen, **dadurch gekennzeichnet, dass** das Grundmaterial zur Herstellung der Substrate des Katalysators USY-Zeolithe aufweist, wobei die USY-Zeolithe dealuminiert werden, sodass die Substrate des Katalysators einen Porendurchmesser an der Oberfläche zwischen 10 und 25 Ängström, bevorzugt zwischen 12 und 20 Ängstrom, besonders bevorzugt im Wesentlichen von 15 Ängstrom aufweisen, und dass das Substrat mit Silicowolframsäure oder Phosphorwolframsäure versehen wird.

15. Verfahren nach einem der Ansprüche 12 bis 14, **dadurch gekennzeichnet, dass** die Herstellung des Katalysators einen oder mehrere der Schritte umfasst:
das Miteinarbeiten organischer Bindemittel, die anschließend rückstandslos ausgebrannt werden, wobei dabei vorzugsweise das organische Bindemittel in die Grundmaterialien, die bevorzugt pulverförmig vorliegen, eingearbeitet und diese anschließend homogen zu einer Grundmasse verknetet werden, wobei als organische Bindemittel bevorzugt eine Dispersion, besonders bevorzugt ein Nano-Dispersion, aus Polymeren, vorzugsweise Polystyrol, und Wasser verwendet werden, wobei vorzugsweise der Polymeranteil kleiner als 5 Gewichtsprozent, besonders bevorzugt kleiner als 1 Gewichtsprozent an der Dispersion beträgt,
das Extrudieren der Pellets bevorzugt unter einem Druck zwischen 5 und 120 bar, besonders bevorzugt zwischen 20 und 100 bar, noch mehr bevorzugt zwischen 40 und 80 bar auf einen Durchmesser zwischen 1 und 8 mm, bevorzugt zwischen 2 und 6 mm, besonders bevorzugt zwischen 3 und 4 mm und/oder eine Länge zwischen 0,25 und 4 cm, bevorzugt zwischen 0,4 und 3 cm, besonders bevorzugt zwischen 0,5 und 2 cm und vorzugsweise die Konditionierung der Pellets bei einer Temperatur zwischen 400 und 1000 °C, bevorzugt zwischen 500 und 750 °C, noch mehr bevorzugt bei 600 °C für einen Zeitraum zwischen 24 und 168 h, bevorzugt zwischen 36 und 96 h, noch mehr bevorzugt für 48h,
Imprägnierung des extrudierten Substrats mit Platin, pro 1000 g Substratmaterial vorzugsweise mit zwischen 0,5 und 3 Litern, bevorzugt 1,5 Liter wässriger Lösung von zwischen 15 und 300 mmol/l, bevorzugt 75 mmol/l H2PtCl6·6H2O, vorzugsweise anschließende Trocknung bei zwischen 60 und 120 °C, bevorzugt 90 °C und vorzugsweise anschließende Kalzinierung für zwischen 6 und 24 Stunden, bevorzugt 12 Stunden bei zwischen 250 und 450 °C, bevorzugt 400 °C, und/oder
Imprägnierung des platindotierten Substrats mit zwischen 0,5 und 3 Litern, bevorzugt 1,5 Litern zwischen 25 und 100 mmol/l, bevorzugt 50 mmol/l wässriger Silicowolframsäure zur oberflächlichen Fixierung des Dehydratationskatalysators, vorzugsweise anschließende Trocknung bei zwischen 60 und 120 °C, vorzugsweise 90 °C und vorzugsweise anschließende Kalzinierung bei zwischen 250 und 450 °C, bevorzugt 350 °C für zwischen 6 und 24 Stunden, bevorzugt 12 Stunden.

## Claims

1. Method for the catalytic conversion of a substance mixture containing glycerol into propanols in a fixed-bed reactor (2), substrates of the catalyst comprising inorganic materials and/or metal oxides, **characterised in that** the substrates have a pore diameter at the surface of between 10 and 25 angstroms, preferably between 12 and 20 angstroms, more preferably 15 angstroms, the catalyst comprising silicotungstic acid or phosphotungstic acid.

2. Method according to claim 1, **characterised in that** the substrates are extrudate pellets and are preferably ceramic; and/or
**characterised in that** the reaction of the substance mixture containing glycerol in the fixed-bed reactor (2), which is preferably single-stage, takes place continuously.

3. Method according to either claim 1 or claim 2, **characterised in that** mixed oxides of silicon, tungsten, zirconium and/or aluminium, preferably zirconium dioxide, aluminosilicate zeolites and/or aluminophosphate zeolites, preferably VFI zeolites and/or VPI-5 zeolites, dealuminated USY zeolites and/or aluminium dioxide are used as the substrate of the catalyst, with the catalyst preferably having platinum doping; and/or
**characterised in that** the substance mixture containing glycerol is crude glycerol, technical glycerol or purified glycerol.

4. Method according to any of the preceding claims, **characterised in that** the method comprises one or more of the following steps:
removing the substance mixture containing glycerol from a storage container (3),
filtering the substance mixture containing glycerol, with undissolved solids and/or diluted accompanying substances contained therein preferably being removed, and/or
mixing the substance mixture containing glycerol with water in the mixer (5), the concentration of the glycerol preferably being adjusted to between 5 and 80 percent, particularly preferably to between 10 and 60 percent, even more preferably to between 15 and 50 percent; and/or
**characterised in that** the method comprises one or more of the following steps:
heating the substance mixture containing glycerol with a heat exchanger (6), preferably an economiser,
adding hydrogen to the substance mixture containing glycerol, preferably in a static mixer (7),
feeding the heat of the substance mixture reacted in the fixed-bed reactor (2) back to the heat exchanger (6) for heat transfer to the substance mixture containing glycerol before the reaction, such that the method is adiabatic.

5. Method according to any of the preceding claims, **characterised in that** hydrogen is recovered from the substance mixture reacted in the fixed-bed reactor (2) by means of a separator apparatus (9), the recovered hydrogen preferably being subsequently recompressed in a compressor (10) and admixed with fresh hydrogen, the hydrogen mixture particularly preferably being used at an earlier point in the method and the recovered hydrogen even more preferably having a proportion of between 50 and 99 percent, preferably between 70 and 97 percent, of the total hydrogen used in the method; and/or
**characterised in that**
propanols, preferably 1-propanol and 2-propanol, are obtained from the substance mixture reacted in the fixed-bed reactor (2), preferably by first removing water from the substance mixture reacted in the fixed-bed reactor (2) via a processing cascade (11) and obtaining pure propanols, the separated process water being admixed with fresh water in a water mixer (12) and the water mixture particularly preferably being used at an earlier point in the method, in particular for mixing in the mixer (5), and the recovered process water even more preferably having a proportion between 80 and 100 percent, preferably between 90 and 100 percent, of the total water used in the method, and/or
long-chain hydrocarbons are built up, preferably oligomerised, from the substance mixture reacted in the fixed-bed reactor (2).

6. Method according to any of the preceding claims, **characterised in that** the reaction in the fixed-bed reactor (2) takes place at a temperature between 150 and 300°C, preferably between 190 and 250°C, particularly preferably between 210 and 230°C; and/or
**characterised in that** the reaction in the fixed-bed reactor (2) takes place at a pressure of between 10 and 100 bar, preferably between 15 and 75 bar, particularly preferably between 25 and 50 bar.

7. Device (1) for the catalytic conversion of a substance mixture containing glycerol, preferably crude glycerol, into propanols in a fixed-bed reactor (2), the device comprising a catalyst and substrates of the catalyst comprising inorganic materials and/or metal oxides, **characterised in that** the substrates have a pore diameter at the surface of between 10 and 25 angstroms, preferably between 12 and 20 angstroms, more preferably substantially 15 angstroms, the substrates preferably being extrudate pellets and preferably being ceramic,
the catalyst comprising silicotungstic acid or phosphotungstic acid.

8. Device (1) according to claim 7, **characterised in that**
the substrates of the catalyst comprise mixed oxides of silicon, tungsten, zirconium and/or aluminium, preferably zirconium dioxide, aluminosilicate zeolites and/or aluminophosphate zeolites, preferably VFI zeolites and/or VPI-5 zeolites, dealuminated USY zeolites and/or aluminium dioxide and preferably have platinum doping, the fixed-bed reactor (2) preferably being suitable for continuous operation.

9. Device (1) according to either claim 7 or claim 8, **characterised in that** the device comprises:
a storage container (3) for the substance mixture containing glycerol,
a filter device (4) for removing undissolved solids and/or unwanted accompanying substances contained in the substance mixture containing glycerol,
a first mixer (5) for diluting the substance mixture containing glycerol with water,
a heat exchanger (6), preferably an economiser, for heating the substance mixture containing glycerol,
a preferably static second mixer (7) for adding hydrogen to the substance mixture containing glycerol, a line (8) for feeding the substance mixture reacted in the fixed-bed reactor back to the heat exchanger,
a separator apparatus (9) for recovering hydrogen from the substance mixture converted in the fixed-bed reactor (2) and preferably a compressor (10) for compressing the recovered hydrogen, and/or
a processing cascade (11) for obtaining the pure propanol fraction and preferably for recovering the process water from the substance mixture reacted in the fixed-bed reactor (2) for mixing with fresh water in the water mixer (12), the water mixture obtained thereby preferably being fed to the mixer (5).

10. Catalyst for the catalytic conversion of a substance mixture containing glycerol into propanols in a fixed-bed reactor (2), substrates of the catalyst comprising inorganic materials and/or metal oxides, **characterised in that** the substrates have a pore diameter at the surface of between 10 and 25 angstroms, preferably between 12 and 20 angstroms, particularly preferably substantially 15 angstroms,
the catalyst comprising silicotungstic acid or phosphotungstic acid.

11. Catalyst according to claim 10, **characterised in that** the substrates are extrudate pellets and are preferably ceramic,
the substrates of the catalyst preferably comprising mixed oxides of silicon, tungsten, zirconium and/or aluminium, preferably zirconium dioxide, aluminosilicate zeolites and/or aluminophosphate zeolites, preferably VFI zeolites and/or VPI-5 zeolites, dealuminated USY zeolites and/or aluminium dioxide and preferably have platinum doping and/or
the catalyst being suitable for continuous operation of the fixed-bed reactor (2).

12. Method for producing a catalyst according to either claim 10 or claim 11 for the catalytic conversion of a substance mixture containing glycerol into propanols in a fixed-bed reactor, substrates of the catalyst comprising inorganic materials and/or metal oxides, **characterised in that** base materials for producing the substrates of the catalyst have a pore diameter at the surface of between 10 and 25 angstroms, preferably between 12 and 20 angstroms, more preferably substantially 15 angstroms, and **in that** the substrate is provided with silicotungstic acid or phosphotungstic acid.

13. Method according to claim 12, **characterised in that** mixed oxides of silicon, tungsten, zirconium and/or aluminium, preferably zirconium dioxide, aluminosilicate zeolites and/or aluminophosphate zeolites, preferably VFI zeolites and/or VPI-5 zeolites, and/or aluminium dioxide can be used as base materials for the substrates.

14. Method for producing a catalyst according to either claim 10 or claim 11 for the catalytic conversion of a substance mixture containing glycerol into propanols in a fixed-bed reactor, the substrates of the catalyst comprising inorganic materials and/or metal oxides, **characterised in that** the base material for producing the substrates of the catalyst comprises USY zeolites, the USY zeolites being dealuminated so that the substrates of the catalyst have a pore diameter at the surface of between 10 and 25 angstroms, preferably between 12 and 20 angstroms, more preferably substantially 15 angstroms, and **in that** the substrate is provided with silicotungstic acid or phosphotungstic acid.

15. Method according to any of claims 12 to 14, **characterised in that** the production of the catalyst comprises one or more of the following steps:
incorporating organic binders which are subsequently burned off without leaving residue, the organic binder preferably being incorporated into the base materials, which are preferably in powder form, and these then being homogeneously kneaded to form a base compound, a dispersion, particularly preferably a nano-dispersion, of polymers, preferably polystyrene, and water preferably being used as the organic binder, the polymer content preferably being less than 5 wt.%, particularly preferably less than 1 wt.%, of the dispersion,
extruding the pellets, preferably under a pressure of between 5 and 120 bar, particularly preferably between 20 and 100 bar, even more preferably between 40 and 80 bar, to a diameter of between 1 and 8 mm, preferably between 2 and 6 mm, particularly preferably between 3 and 4 mm, and/or a length between 0.25 and 4 cm, preferably between 0.4 and 3 cm, particularly preferably between 0.5 and 2 cm, and preferably conditioning the pellets at a temperature between 400 and 1000°C, preferably between 500 and 750°C, even more preferably at 600°C, for a period of between 24 and 168 h, preferably between 36 and 96 h, even more preferably for 48 h,
impregnating the extruded substrate with platinum, per 1000 g of substrate material, preferably with between 0.5 and 3 litres, preferably 1.5 litres, of aqueous solution of between 15 and 300 mmol/l, preferably 75 mmol/l, of H2PtCI6 · 6H2O, preferably subsequently drying at between 60 and 120°C, preferably 90°C, and preferably subsequently carrying out calcination for between 6 and 24 hours, preferably 12 hours, at between 250 and 450°C, preferably 400°C, and/or
impregnating the platinum-doped substrate with between 0.5 and 3 litres, preferably 1.5 litres, between 25 and 100 mmol/l, preferably 50 mmol/l, of aqueous silicotungstic acid for surface fixation of the dehydration catalyst, preferably subsequently drying at between 60 and 120°C, preferably 90°C, and preferably subsequently carrying out calcination at between 250 and 450°C, preferably 350°C, for between 6 and 24 hours, preferably 12 hours.

## Revendications

1. Procédé pour la conversion catalytique d'un mélange de substances contenant de la glycérine en propanols dans un réacteur à lit fixe (2), où des substrats du catalyseur comportent des matériaux inorganiques et/ou des oxydes métalliques, **caractérisé en ce que** les substrats comportent un diamètre de pores à la surface entre 10 et 25 angströms, de préférence entre 12 et 20 angströms, de manière particulièrement préférée de 15 angströms, où le catalyseur comporte de l'acide silicotungstique ou de l'acide phosphotungstique.

2. Procédé selon la revendication 1, **caractérisé en ce que** les substrats sont des granulés de produit d'extrusion et sont de préférence céramiques; et/ou
**caractérisé en ce que** la réaction du mélange de substances contenant de la glycérine dans le réacteur à lit fixe (2), qui est de préférence à étage unique, a lieu en continu.

3. Procédé selon l'une quelconque des revendications 1 ou 2, **caractérisé en ce que** comme substrat du catalyseur sont utilisés des oxydes mixtes de silicium, de tungstène, de zirconium et/ou d'aluminium, de préférence du dioxyde de zirconium, des zéolithes d'aluminosilicate et/ou d'aluminophosphate, de préférence des zéolithes VFI et/ou VPI-5, des zéolithes USY désaluminées et/ou du dioxyde d'aluminium, où de préférence le catalyseur comporte un dopage au platine; et/ou
**caractérisé en ce que** le mélange de substances contenant de la glycérine est de la glycérine brute, de la glycérine technique ou de la glycérine purifiée.

4. Procédé selon l'une des revendications précédentes, **caractérisé en ce que** le procédé comporte une ou plusieurs des étapes suivantes:
le prélèvement du mélange de substances contenant de la glycérine d'un récipient de stockage (3),
la filtration du mélange de substances contenant de la glycérine, où de préférence les matières solides non dissoutes et/ou les impuretés diluées contenues dans celui-ci sont retirées, et/ou
le mélange du mélange de substances contenant de la glycérine avec de l'eau dans le mélangeur (5), où de préférence la concentration de la glycérine est ajustée à entre 5 et 80 pour cent, de manière particulièrement préférée entre 10 et 60 pour cent, de préférence encore entre 15 et 50 pour cent; et/ou
**caractérisé en ce que** le procédé comporte une ou plusieurs des étapes suivantes:
chauffage du mélange de substances contenant de la glycérine avec un échangeur de chaleur (6), de préférence un économiseur,
addition d'hydrogène au mélange de substances contenant de la glycérine, de préférence dans un mélangeur statique (7),
recyclage de la chaleur du mélange de substances mis à réagir dans le réacteur à lit fixe (2) vers l'échangeur de chaleur (6) pour transmettre la chaleur au mélange de substances contenant de la glycérine avant la réaction, de sorte que le procédé a lieu de manière adiabatique.

5. Procédé selon l'une des revendications précédentes, **caractérisé en ce que** l'hydrogène est récupéré à partir du mélange de substances mis à réagir dans le réacteur à lit fixe (2) par l'intermédiaire d'un dispositif de séparation (9), où de préférence l'hydrogène récupéré est ensuite recomprimé dans un compresseur (10) et mélangé à un hydrogène frais, où le mélange d'hydrogène est de manière particulièrement préférée utilisé à un stade antérieur dans le procédé et de manière préférée encore l'hydrogène récupéré a une proportion entre 50 et 99 pour cent, de préférence entre 70 et 97 pour cent de l'hydrogène total utilisé dans le procédé; et/ou
**caractérisé en ce que**
des propanols, de préférence du 1-propanol et aussi du 2-propanol, sont obtenus à partir du mélange de substances mis à réagir dans le réacteur à lit fixe (2), de préférence **en ce que** l'eau est d'abord retirée du mélange de substances mis à réagir dans le réacteur à lit fixe (2) par le biais d'une cascade de traitement (11) et des propanols purs sont obtenus, où l'eau de traitement séparée est mélangée à une eau fraîche dans un mélangeur d'eau (12) et le mélange d'eau est utilisé de manière particulièrement préférée dans le procédé à un stade antérieur, en particulier pour le mélange dans le mélangeur (5), et de préférence encore l'eau de procédé récupérée a une proportion entre 80 et 100 pour cent, de préférence entre 90 et 100 pour cent, de l'eau totale utilisée dans le procédé et/ou
à partir du mélange de substances mis à réagir dans le réacteur à lit fixe (2), des hydrocarbures à chaîne plus longue sont formés, de préférence sont oligomérisés.

6. Procédé selon l'une des revendications précédentes, **caractérisé en ce que** la réaction dans le réacteur à lit fixe (2) a lieu à une température entre 150 et 300°C, de préférence entre 190 et 250°C, de manière particulièrement préférée entre 210 et 230°C; et/ou
**caractérisé en ce que** la réaction dans le réacteur à lit fixe (2) a lieu à une pression entre 10 et 100 bars, de préférence entre 15 et 75 bars, de manière particulièrement préférée entre 25 et 50 bars.

7. Dispositif (1) pour la conversion catalytique d'un mélange de substances contenant de la glycérine, de préférence de la glycérine brute, en propanols dans un réacteur à lit fixe (2), où le dispositif comporte un catalyseur et des substrats du catalyseur comportent des matériaux inorganiques et/ou des oxydes métalliques, **caractérisé en ce que** les substrats comportent un diamètre de pores à la surface entre 10 et 25 angströms, de préférence entre 12 et 20 angströms, de manière particulièrement préférée sensiblement de 15 angströms, où les substrats sont de préférence des granulés de produit d'extrusion et sont de préférence céramiques, où
le catalyseur comporte de l'acide silicotungstique ou de l'acide phosphotungstique.

8. Dispositif (1) selon la revendication 7, **caractérisé en ce que** les substrats du catalyseur comportent des oxydes mixtes de silicium, de tungstène, de zirconium et/ou d'aluminium, de préférence du dioxyde de zirconium, des zéolithes d'aluminosilicate et/ou d'aluminophosphate, de préférence des zéolithes VFI et/ou VPI-5, des zéolithes USY désaluminées et/ou du dioxyde d'aluminium et de préférence un dopage au platine, où de préférence le réacteur à lit fixe (2) convient au fonctionnement continu.

9. Dispositif (1) selon l'une des revendications 7 ou 8, **caractérisé en ce que** le dispositif comporte:
un récipient de stockage (3) pour le mélange de substances contenant de la glycérine,
un dispositif de filtration (4) pour retirer les matières solides non dissoutes et/ou les impuretés indésirables contenues dans le mélange de substances contenant de la glycérine,
un premier mélangeur (5) pour diluer le mélange de substances contenant de la glycérine avec de l'eau,
un échangeur de chaleur (6), de préférence un économiseur, pour chauffer le mélange de substances contenant de la glycérine,
un deuxième mélangeur (7) de préférence statique pour ajouter au mélange de substances contenant de la glycérine de l'hydrogène,
une conduite (8) pour le recyclage vers l'échangeur de chaleur du mélange de substances mis à réagir dans le réacteur à lit fixe,
un dispositif de séparation (9) pour récupérer l'hydrogène du mélange de substances mis à réagir dans le réacteur à lit fixe (2) et de préférence un compresseur (10) pour comprimer l'hydrogène récupéré, et/ou
une cascade de traitement (11) pour obtenir la fraction de propanol pur et de préférence pour récupérer l'eau de procédé du mélange de substances mis à réagir dans le réacteur à lit fixe (2) pour le mélange avec de l'eau fraîche dans le mélangeur d'eau (12), où le mélange d'eau ainsi obtenu est de préférence envoyé au mélangeur (5).

10. Catalyseur pour la conversion catalytique d'un mélange de substances contenant de la glycérine en propanols dans un réacteur à lit fixe (2), où des substrats du catalyseur comportent des matériaux inorganiques et/ou des oxydes métalliques, **caractérisé en ce que** les substrats comportent un diamètre de pores à la surface entre 10 et 25 angströms, de préférence entre 12 et 20 angströms, de manière particulièrement préférée sensiblement de 15 angströms, où le catalyseur comporte de l'acide silicotungstique ou de l'acide phosphotungstique.

11. Catalyseur selon la revendication 10, **caractérisé en ce que** les substrats sont des granulés de produit d'extrusion et sont de préférence céramiques, où
les substrats du catalyseur comportent de préférence des oxydes mixtes de silicium, de tungstène, de zirconium et/ou d'aluminium, de préférence du dioxyde de zirconium, des zéolithes d'aluminosilicate et/ou d'aluminophosphate, de préférence des zéolithes VFI et/ou VPI-5, des zéolithes USY désaluminées et/ou du dioxyde d'aluminium et de préférence un dopage au platine et/ou
le catalyseur convient au fonctionnement continu du réacteur à lit fixe (2).

12. Procédé pour la production d'un catalyseur selon l'une des revendications 10 ou 11 pour la conversion catalytique d'un mélange de substances contenant de la glycérine en propanols dans un réacteur à lit fixe, où des substrats du catalyseur comportent des matériaux inorganiques et/ou des oxydes métalliques, **caractérisé en ce que** les matériaux de base pour la production des substrats du catalyseur comportent un diamètre de pores à la surface entre 10 et 25 angströms, de préférence entre 12 et 20 angströms, de manière particulièrement préférée sensiblement de 15 angströms, et **en ce que** le substrat est pourvu d'acide silicotungstique ou d'acide phosphotungstique.

13. Procédé selon la revendication 12, **caractérisé en ce que** comme matériaux de base pour les substrats sont utilisés des oxydes mixtes de silicium, de tungstène, de zirconium et/ou d'aluminium, de préférence du dioxyde de zirconium, des zéolithes d'aluminosilicate et/ou d'aluminophosphate, de préférence des zéolithes VFI et/ou VPI-5, et/ou du dioxyde d'aluminium.

14. Procédé pour la production d'un catalyseur selon l'une des revendications 10 ou 11 pour la conversion catalytique d'un mélange de substances contenant de la glycérine en propanols dans un réacteur à lit fixe, où des substrats du catalyseur comportent des matériaux inorganiques et/ou des oxydes métalliques, **caractérisé en ce que** le matériau de base pour la production des substrats du catalyseur comporte des zéolithes USY, où les zéolithes USY sont désaluminées, de sorte que les substrats du catalyseur comportent un diamètre de pores à la surface entre 10 et 25 angströms, de préférence entre 12 et 20 angströms, de manière particulièrement préférée sensiblement de 15 angströms, et **en ce que** le substrat est pourvu d'acide silicotungstique ou d'acide phosphotungstique.

15. Procédé selon l'une des revendications 12 à 14, **caractérisé en ce que** la production du catalyseur comporte une ou plusieurs des étapes:
incorporation conjointe de liants organiques, qui sont ensuite grillés sans résidu, où de préférence le liant organique est alors incorporé dans les matériaux de base qui sont de préférence présents sous forme de poudre, et ceux-ci sont ensuite malaxés de manière homogène en une masse de base, où de préférence une dispersion, de manière particulièrement préférée une nano-dispersion, de polymères, de préférence de polystyrène, et de l'eau sont utilisées comme liants organiques, où de préférence la teneur en polymères est inférieure à 5 pour cent en poids, de manière particulièrement préférée inférieure à 1 pour cent en poids de la dispersion,
extrusion des granulés de préférence sous une pression entre 5 et 120 bars, de manière particulièrement préférée entre 20 et 100 bars, de manière préférée encore entre 40 et 80 bars jusqu'à un diamètre entre 1 et 8 mm, de préférence entre 2 et 6 mm, de manière particulièrement préférée entre 3 et 4 mm et/ou une longueur entre 0,25 et 4 cm, de préférence entre 0,4 et 3 cm, de manière particulièrement préférée entre 0,5 et 2 cm et de préférence conditionnement des granulés à une température entre 400 et 1000°C, de préférence entre 500 et 750°C, de préférence encore à 600°C pendant une durée entre 24 et 168 h, de préférence entre 36 et 96 h, de préférence encore pendant 48 h,
imprégnation du substrat extrudé avec du platine, pour 1000 g de matériau de substrat de préférence avec entre 0,5 et 3 litres, de préférence 1,5 litre de solution aqueuse d'entre 15 et 300 mmol/l, de préférence 75 mmol/l de H2PtCl6.6H2O, de préférence séchage ultérieur à entre 60 et 120°C, de préférence 90°C et de préférence calcination ultérieure pendant entre 6 et 24 heures, de préférence 12 heures à entre 250 et 450°C, de préférence 400°C, et/ou
imprégnation du substrat dopé au platine avec entre 0,5 et 3 litres, de préférence 1,5 litre d'acide silicotungstique aqueux à entre 25 et 100 mmol/l, de préférence 50 mmol/l pour la fixation superficielle du catalyseur de déshydratation, de préférence séchage ultérieur à entre 60 et 120°C, de préférence 90°C et de préférence calcination ultérieure à entre 250 et 450°C, de préférence 350°C pendant entre 6 et 24 heures, de préférence 12 heures.
